# EUROPEAN PATENT APPLICATION

(11) **EP 4 755 884 A1**
(43) Date of publication of application: **10.06.2026**
(21) Application number: 24848009.7
(22) Date of filing: 15.07.2024
(51) Int. Cl.: C07D 471/04, C07D 513/04, C07D 401/12, A61K 31/4162, A61P 35/00

(54) **CRYSTAL FORM OF BEZUCLASTINIB, PREPARATION METHOD THEREFOR AND USE THEREOF**

(30) Priority: 28.07.2023 CN 202310943067; 20.09.2023 CN 202311218559; 23.10.2023 CN 202311376497; 27.11.2023 CN 202311596731
(71) Applicant: Cogent Biosciences, Inc., Waltham, Massachusetts 02451 (US)
(72) Inventor: ZHANG, Yuxing, Suzhou, Jiangsu 215123 (CN); QIAN, Jiale, Suzhou, Jiangsu 215123 (CN); MENG, Liping, Suzhou, Jiangsu 215123 (CN)
(74) Representative: Conn, Anne-Marie
(86) International application number: PCT/CN2024/105476
(87) International publication number: WO 2025/026050

(57) **Abstract**

The present invention relates to a crystal form of Bezuclastinib and a preparation method therefor, a pharmaceutical composition comprising the crystal form, and a use of the crystal form in preparation of drugs for inhibiting KIT D816V mutations and drugs for treating gastrointestinal stromal tumor and progressive systemic mastocytosis.

## Description

### TECHNICAL FIELD

The present invention relates to the field of crystal chemistry, and in particular, to the crystal form of Bezuclastinib, a preparation method therefor and a use thereof.

### BACKGROUND

Bezuclastinib is an oral selective tyrosine kinase receptor inhibitor (TKI) developed by Cogent Biosciences. Bezuclastinib can inhibit KIT D816V mutations, and it is used to treat gastrointestinal stromal tumor (GIST) and progressive systemic mastocytosis (AdvSM) with positive clinical results.

The chemical name of Bezuclastinib is 3,4-dimethyl-N-(2-phenyl-1H-pyrrolo[2,3-b]pyridin-5-yl)-1H-pyrazol-5-carboxamide (hereinafter referred to as "compound I"), and its structural formula is as follows:

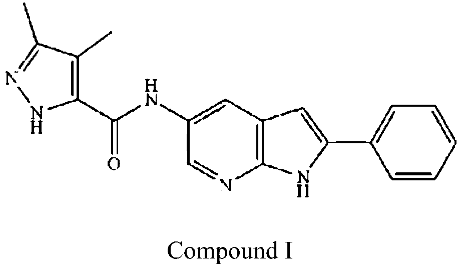

WO2014100620A3 and WO2021222442A1 disclose methods for synthesizing compound I, but do not disclose the crystal form of compound I.

Crystal is a solid in which the molecules of a compound are arranged in a three-dimensional order in a microstructure to form a crystal lattice. Polymorphism refers to the phenomenon that a compound has multiple crystal forms. The compound may exist in one or more crystal forms, but its existence and characteristics cannot be predicted specifically. Different crystal forms of active pharmaceutical ingredients (APIs) have different physical and chemical properties, which may lead to different dissolution and absorption of the drug in vivo, and then affect the clinical efficacy of the drug to some extent. Especially for some insoluble oral solid or semi-solid preparations, the crystal form is very important to the product performance. In addition, the physical and chemical properties of crystal form are very important to the production process. Therefore, polymorphism is an important aspect of drug research and drug quality control.

In order to develop drugs containing compound I, new crystal forms that meet the medical needs are needed. The inventors of the present application unexpectedly found the crystal form of compound I provided by the present invention, which has advantages in at least one of solubility, hygroscopicity, purification effect, stability, adhesiveness, compressibility, fluidity, dissolution in vitro and in vivo, biological effectiveness, etc. In particular, it has good stability, low hygroscopicity, and is suitable for use in medicine, which is of great significance for the development of drugs containing compound I.

### SUMMARY OF THE INVENTION

The present invention provides a crystalline solid of compound I, a preparation method therefor and a pharmaceutical composition comprising the crystalline solid.

According to the purpose of the present invention, the present invention provides a crystalline solid of compound I.

According to the purpose of the present invention, the present invention provides crystal form CSI of compound I (hereinafter referred to as "crystal form CSI").

In an aspect, the X-ray powder diffraction pattern by Cu-Kα radiation of crystal form CSI has a characteristic peak at 1, 2 or 3 of diffraction angles 2θ of 7.5°±0.2°, 13.7°±0.2°, 16.8°±0.2°. Preferably, the X-ray powder diffraction pattern of crystal form CSI has characteristic peaks at diffraction angles 2θ of 7.5°±0.2°, 13.7°±0.2°, 16.8°±0.2°.

Further, the X-ray powder diffraction pattern by Cu-Kα radiation of crystal form CSI has a characteristic peak at 1, 2 or 3 of diffraction angles 20 of 13.0°±0.2°, 15.1°±0.2°, 18.9°±0.2°; preferably, the X-ray powder diffraction pattern of crystal form CSI has characteristic peaks at diffraction angles 20 of 13.0°±0.2°, 15.1°±0.2°, 18.9°±0.2°.

Further, the X-ray powder diffraction pattern by Cu-Kα radiation of crystal form CSI has a characteristic peak at 1, 2 or 3 of diffraction angles 20 of 22.8°±0.2°, 23.6°±0.2°, 25.6°±0.2°; preferably, the X-ray powder diffraction pattern of crystal form CSI has characteristic peaks at diffraction angles 20 of 22.8°±0.2°, 23.6°±0.2°, 25.6°±0.2°.

In another aspect, the X-ray powder diffraction pattern by Cu-Kα radiation of crystal form CSI has a characteristic peak at 1, 2, 3, 4, 5, 6, 7, 8 or 9 of diffraction angles 2θ of 7.5°±0.2°, 13.7°±0.2°, 14.7°±0.2°, 16.8°±0.2°, 13.0°±0.2°, 15.1°±0.2°, 18.9°±0.2°, 22.8°±0.2°, 23.6°±0.2°, and 25.6°±0.2°.

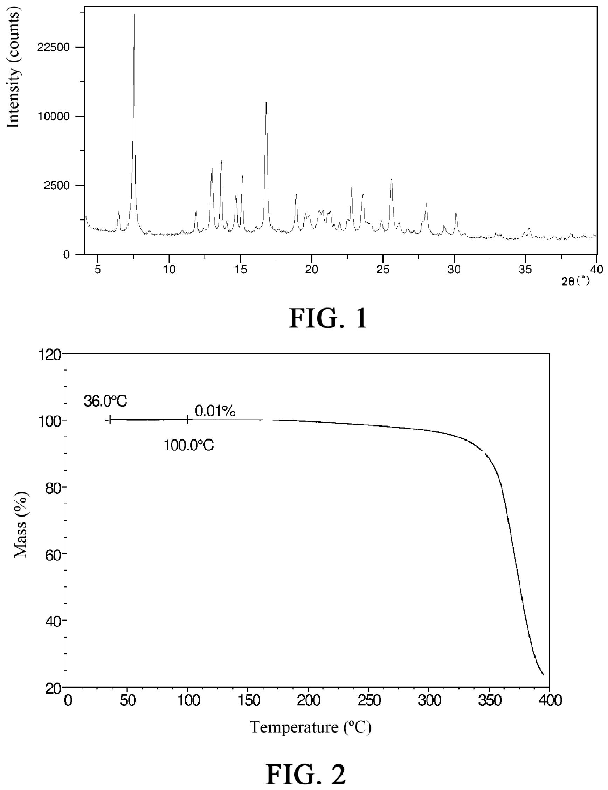

Without limitation, the X-ray powder diffraction pattern by Cu-Kα radiation of crystal form CSI is substantially as shown in Fig. 1.

Without limitation, the thermogravimetric analysis diagram of the crystal form CSI is substantially as shown in Fig. 2, with a mass loss of about 0.01% when heated to 100°C.

Without limitation, the crystal form CSI is anhydrous.

According to the purpose of the present invention, the present invention provides crystal form CSII of compound I (hereinafter referred to as "crystal form CSII").

In an aspect, the X-ray powder diffraction pattern by Cu-Kα radiation of crystal form CSII has a characteristic peak at 1, 2 or 3 of diffraction angles 20 of 8.6°±0.2°, 12.5°±0.2°, 22.0°±0.2°. Preferably, the X-ray powder diffraction pattern of crystal form CSII has characteristic peaks at diffraction angles 2θ of 8.6°±0.2°, 12.5°±0.2°, 22.0°±0.2°.

Further, the X-ray powder diffraction pattern by Cu-Kα radiation of crystal form CSII has a characteristic peak at 1, 2 or 3 of diffraction angles 2θ of 7.9°±0.2°, 10.9°±0.2°, 26.1°+0.2°; preferably, the X-ray powder diffraction pattern of crystal form CSII has characteristic peaks at diffraction angles 2θ of 7.9°±0.2°, 10.9°±0.2°, 26.1°±0.2°.

Further, the X-ray powder diffraction pattern by Cu-Kα radiation of crystal form CSII has a characteristic peak at 1 or 2 of diffraction angles 20 of 18.1°±0.2° and 28.8°±0.2°; preferably, the X-ray powder diffraction pattern of crystal form CSII has characteristic peaks at diffraction angles 20 of 18.1°±0.2° and 28.8°±0.2°.

In another aspect, the X-ray powder diffraction pattern by Cu-Kα radiation of crystal form CSII has a characteristic peak at 1, 2, 3, 4, 5, 6, 7, 8 or 9 of diffraction angles 2θ of 8.6°±0.2°, 12.5°±0.2°, 22.0°±0.2°, 7.9°±0.2°, 10.9°±0.2°, 26.1°±0.2°, 18.1°±0.2°, 28.8°±0.2°, 17.1°±0.2°.

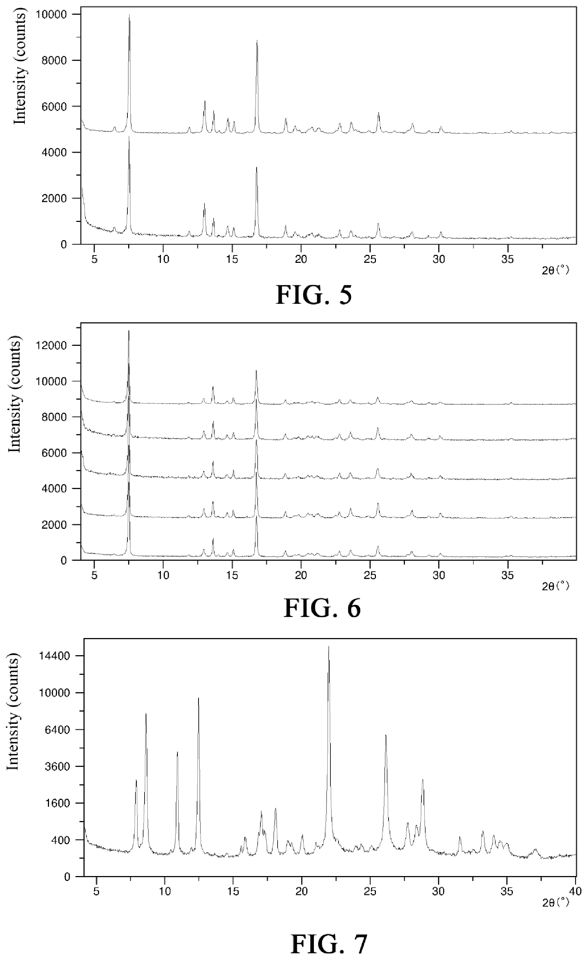

Without limitation, the X-ray powder diffraction pattern by Cu-Kα radiation of crystal form CSII is substantially as shown in Fig. 7.

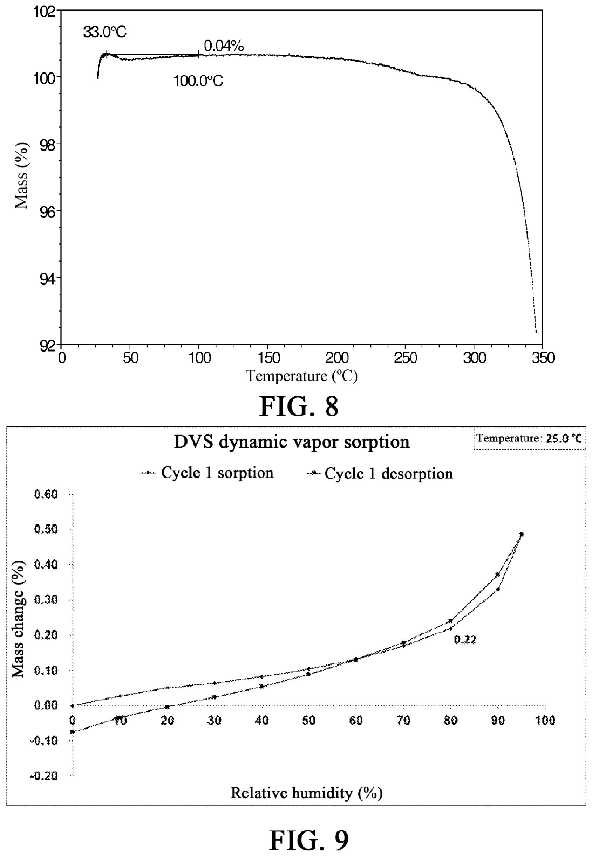

Without limitation, the thermogravimetric analysis diagram of the crystal form CSII is substantially as shown in Fig. 8, with a mass loss of about 0.04% when heated to 100°C.

Without limitation, the crystal form CSII is anhydrous.

According to the purpose of the present invention, the present invention provides crystal form CSIII of compound I (hereinafter referred to as "crystal form CSIII").

In an aspect, the X-ray powder diffraction pattern by Cu-Kα radiation of crystal form CSIII has a characteristic peak at 1, 2 or 3 of diffraction angles 20 of 7.4°±0.2°, 11.5°±0.2°, 16.2°±0.2°. Preferably, the X-ray powder diffraction pattern of crystal form CSIII has characteristic peaks at diffraction angles 20 of 7.4°±0.2°, 11.5°±0.2°, 16.2°±0.2°.

Further, the X-ray powder diffraction pattern by Cu-Kα radiation of crystal form CSIII has a characteristic peak at 1, 2 or 3 of diffraction angles 20 of 13.4°±0.2°, 14.9°±0.2°, 18.0°±0.2°; preferably, the X-ray powder diffraction pattern of crystal form CSIII has characteristic peaks at diffraction angles 20 of 13.4°±0.2°, 14.9°±0.2°, 18.0°±0.2°.

Further, the X-ray powder diffraction pattern by Cu-Kα radiation of crystal form CSIII has a characteristic peak at a diffraction angle 2θ of 13.7°±0.2°.

In another aspect, the X-ray powder diffraction pattern by Cu-Kα radiation of crystal form CSIII has a characteristic peak at 1, 2, 3, 4, 5, 6, 7, 8 or 9 of diffraction angles 2θ of 7.4°±0.2°, 11.5°±0.2°, 16.2°±0.2°, 13.4°±0.2°, 14.9°±0.2°, 18.0°±0.2°, 13.7°±0.2°, 5.7°±0.2°, 8.9°±0.2°, 11.0°±0.2°, 17.2°±0.2°, and 22.4°±0.2°.

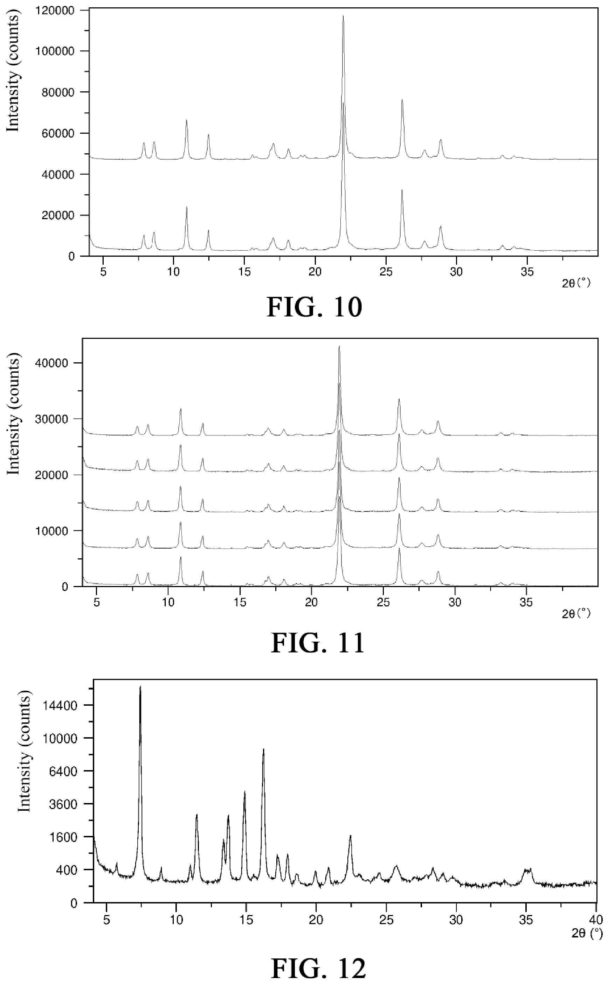

Without limitation, the X-ray powder diffraction pattern by Cu-Kα radiation of crystal form CSIII is substantially as shown in Fig. 12.

According to the purpose of the present invention, the present invention provides a preparation method for the crystal form CSIII of compound I: dissolving compound I in a mixed solvent of dimethyl sulfoxide and water, filtering the mixture, and adding the filtrate into acetonitrile with stirring to obtain the crystal form CSIII.

According to the purpose of the present invention, the present invention provides a pharmaceutical composition, which comprises a therapeutically effective amount of the crystal form CSI, crystal form CSII or crystal form CSIII of compound I, and a pharmaceutically acceptable excipient.

According to the purpose of the present invention, the present invention provides use of the crystal form CSI, crystal form CSII or crystal form CSIII of compound I in preparation of KIT D816V inhibitor drugs.

According to the purpose of the present invention, the present invention provides use of the crystal form CSI, crystal form CSII or crystal form CSIII of compound I in preparation of drugs for treating gastrointestinal stromal tumor and progressive systemic mastocytosis.

Crystal forms CSI, CSII and CSIII provided by the present invention have the following advantages:
(1) Crystal form CSI, crystal form CSII and crystal form CSIII have low hygroscopicity.

On the one hand, high hygroscopicity easily leads to chemical degradation and crystal transformation of APIs, thus affecting the physical and chemical stability of APIs. In addition, high hygroscopicity will reduce the fluidity of APIs, thus affecting the processing process of APIs. On the other hand, drugs with high hygroscopicity need to maintain low humidity during production and storage, which puts forward higher requirements for production and requires high costs. More importantly, high hygroscopicity is easy to cause changes in the content of active ingredients in drugs and affect the quality of drugs.

Crystal form CSI, crystal form CSII and crystal form CSIII provided by the present invention have low hygroscopicity, impose no harsh requirements on the production and storage of drugs, and reduce the costs of drug production, storage and quality control, which is of great economic value.

(2) Crystal form CSI, crystal form CSII and crystal form CSIII have good stability.

Crystal form CSI, crystal form CSII and crystal form CSIII can remain stable for at least 3 months at 40°C/75%RH and 60°C/75%RH, and their purity remains substantially unchanged, which shows that the three crystal forms have good stability under accelerated conditions and more stringent conditions.

Also, form CSI, crystal form CSII and crystal form CSIII have good humidity stability. Crystal forms CSI, CSII and CSIII do not change in crystal form after being circulated once at 0%RH-95%RH-0%RH.

The high temperature and high humidity caused by seasonal differences, climate differences in different regions and environmental factors will affect the storage, transportation and production of APIs and preparations. Therefore, the stability of APIs and preparations under accelerated conditions and more stringent conditions is very important for drugs. Crystal form CSI, crystal form CSII and crystal form CSIII have better stability under harsh conditions, which is conductive to avoiding the influence on drug quality due to crystal transformation or purity decline during drug storage.

The good physical and chemical stability of the crystal form of APIs can ensure that the drug will not undergo crystal transformation during production and storage, and substantially no impurities will be produced. Crystal form CSI, crystal form CSII and crystal form CSIII have good physical and chemical stability, which ensures the consistent and controllable quality of APIs and preparations, and reduces drug quality changes, bioavailability changes and toxic and side effects caused by crystal form changes or impurities.

### DESCRIPTION OF DRAWINGS

Fig. 1 shows XRPD of crystal form CSI.
Fig. 2 shows TGA of crystal form CSI.
Fig. 3 shows TGA of crystal form CSI.
Fig. 4 shows DVS of crystal form CSI.
Fig. 5 shows comparison of XRPD of crystal form CSI before and after DVS.
Fig. 6 shows comparison of XRPD of crystal form CSI stored under different conditions (from top to bottom in sequence: before storage, after storage at 40°C/75%RH for 3 months in a state of being exposed to air, after storage at 40°C/75%RH for 3 months in a sealed state (with desiccant), after storage at 60°C/75%RH for 3 months in a state of being exposed to air, after storage at 60°C/75%RH for 3 months in a sealed state (with desiccant).
Fig. 7 shows XRPD of crystal form CSII.
Fig. 8 shows TGA of crystal form CSII.
Fig. 9 shows DVS of crystal form CSII.
Fig. 10 shows comparison of XRPD of crystal form CSII before and after DVS.
Fig. 11 shows comparison of XRPD of crystal form CSII stored under different conditions (from top to bottom in sequence: before storage, after storage at 40°C/75%RH for 3 months in a state of being exposed to air, after storage at 40°C/75%RH for 3 months in a sealed state (with desiccant), after storage at 60°C/75%RH for 3 months in a state of being exposed to air, after storage at 60°C/75%RH for 3 months in a sealed state (with desiccant).
Fig. 12 shows XRPD of crystal form CSIII.
Fig. 13 shows DVS of crystal form CSIII.
Fig. 14 shows comparison of XRPD of crystal form CSIII before and after DVS.
Fig. 15 shows comparison of XRPD of crystal form CSIII stored under different conditions (from top to bottom in sequence: before storage, after storage at 40°C/75%RH for 3 months in a state of being exposed to air, after storage at 40°C/75%RH for 3 months in a sealed state (with desiccant), after storage at 60°C/75%RH for 3 months in a state of being exposed to air, after storage at 60°C/75%RH for 3 months in a sealed state (with desiccant).

### DETAILED DESCRIPTION

The present invention will be described in detail with reference to the following examples, which describe in detail the preparation and use methods of the crystal forms of the present invention. It is obvious to those skilled in the art that many changes to both materials and methods can be implemented without departing from the scope of the present invention.

The abbreviations used in the present invention are explained as follows:
XRPD: X-ray powder diffraction
TGA: Thermogravimetric analysis
DVS: Dynamic vapor sorption
HPLC: High performance liquid chromatography
¹HNMR: H nuclear magnetic resonance
RH: Relative humidity

### Instruments and methods used to acquire data:

The XRPD diagram of the present invention is acquired on a Bruker D8 ADVANCE X-ray powder diffractometer. The method parameters of X-ray powder diffraction according to the present invention are as follows:
X-ray source: Cu, Kα
Kα1 (Å): 1.54060; Ka2 (Å): 1.54439
Kα2/Ka1 intensity ratio: 0.50
Voltage: 40 kV
Current: 40 mA
Scanning range: from 4.0 to 40.0°.

The TGA diagram of the present invention is acquired on TA Q500. The method parameters of thermogravimetric analysis (TGA) according to the present invention are as follows:
Scanning rate: 10°C/ min
Protective gas: N₂

The DVS diagram of the present invention is acquired on an Intrinsic dynamic vapor sorption instrument manufactured by SMS (Surface Measurement Systems Ltd.). The instrument control software is DVS-Intrinsic control software. The method parameters of the dynamic vapor sorption instrument are as follows:
Temperature: 25°C
Carrier gas, flow rate: N₂, 200 mL/min.
Relative humidity range: 0%RH-95%RH

The ¹H NMR data of the present invention are acquired from a Bruker Avance II DMX 400M HZ nuclear magnetic resonance spectrometer. 1-5 mg of sample is weighed, dissolved with 0.5 mL of deuterated dimethyl sulfoxide to prepare a 2-10 mg/mL solution.

The methods for detecting substances related to the present invention are shown in Table 1.

**Table 1**

| HPLC | Agilent 1260 | |
|---|---|---|
| Chromatographic column | Waters Xbridge C18, 4.6×150 mm, 5 µm | |
| Mobile phase | A: 0.1% aqueous trifluoroacetic acid solution | |
| | B: 0.1% trifluoroacetic acid solution in acetonitrile | |

| | Time (min) | %B |
|---|---|---|
| Gradient | 0.0 | 20 |
| | 12.0 | 60 |
| | 15.0 | 80 |
| | 15.1 | 20 |
| | 18.0 | 20 |
| Flow rate | 1.5 mL/min | |
| Injection volume | 5 µL | |
| Detection wavelength | 338 nm | |
| Column temperature | 40°C | |
| Sample tray temperature | Room temperature | |
| Diluent | Acetonitrile : dimethyl sulfoxide = 5:1 | |

In the present invention, the "crystalline solid" refers to a solid substance with different molecular arrangements and/or conformations in a crystal lattice.

The "anhydrous substance" refers to a crystalline substance that does not contain crystallization water or crystallization solvent.

The "stirring" is completed by conventional methods in the art, such as magnetic stirring or mechanical stirring, and the stirring speed is 50-1800 rpm, wherein the magnetic stirring is preferably at 300-900 rpm and the mechanical stirring is preferably at 100-300 rpm.

The "separation" is accomplished by conventional methods in the art, such as centrifugation or filtration. The "centrifugation" is operated as follows: put the sample to be separated in a centrifuge tube, and centrifuge it at a speed of 10000 rpm until all the solids sink to the bottom of the centrifuge tube.

The "drying" is completed by conventional methods in the art, such as vacuum drying, blast drying or natural air drying. The drying temperature may be room temperature or higher, preferably room temperature to about 60°C, or 50°C, or 40°C. The drying time can be 0.5-48 h, or overnight. Drying is carried out in a fume hood, a blast oven or a vacuum oven. The "room temperature" is not a specific temperature value, but refers to a temperature range of 10-30°C.

The "volatilization" is accomplished by conventional methods in the art, such as slow volatilization or rapid volatilization. Slow volatilization involves sealing the container with sealing film, punching holes thereon and allowing it to stand for volatilization; rapid volatilization involves leaving the container open for evaporation.

The "characteristic peak" refers to representative diffraction peaks used to identify crystals. When Cu-Kα radiation is used for testing, the peak position can usually have an error of ±0.2°.

In the present invention, "crystal" or "crystal form" can be characterized by X-ray powder diffraction. Those skilled in the art can understand that the X-ray powder diffraction pattern is affected and changed by the conditions of the instrument, the preparation of the sample and the purity of the sample. The relative intensity of diffraction peak in X-ray powder diffraction pattern may also change with the change of experimental conditions, so the intensity of diffraction peak cannot be used as the only or decisive factor to judge the crystal form. In fact, the relative intensity of diffraction peak in X-ray powder diffraction pattern is related to the preferred orientation of crystal, and the intensity of diffraction peak shown in the present invention is illustrative and not for absolute comparison. Therefore, it can be understood by those skilled in the art that the X-ray powder diffraction patterns of the crystal forms protected by the present invention need not be exactly the same as the X-ray powder diffraction patterns in the examples referred to herein, and any crystal form with the same or similar X-ray powder diffraction pattern as the characteristic peaks in these patterns belongs to the scope of the present invention. Those skilled in the art can compare the X-ray powder diffraction patterns listed in the present invention with the X-ray powder diffraction patterns of an unknown crystal form to confirm whether the two groups of patterns reflect the same or different crystal forms.

In some embodiments, the crystal forms CSI, CSII and CSIII of the present invention are pure, and are substantially not mixed with any other crystal forms. In the present invention, "substantially not", when used to refer to a new crystal form, means that this crystal form contains less than 20% (by weight) of other crystal forms, especially less than 10% (by weight) of other crystal forms, further less than 5% (by weight) of other crystal forms, and further less than 1% (by weight) of other crystal forms.

The term "about" in the present invention, when used to refer to measurable values, such as mass, time, temperature, means that there can be a certain floating range around specific values, which can be ±10%, +5%, +1%, +0.5%, or ±0.1%.

Unless otherwise specified, the following examples are all operated at room temperature.

According to the present invention, the compound I as a starting material includes, but is not limited to, solid form (crystalline or amorphous), oily form, liquid form and solution. Preferably, the compound I as the starting material is in solid form.

The compound I used in the following examples can be prepared according to the prior art, for example, according to the method described in the document WO2014100620A3.

### Example 1: Preparation method for crystal form CSI

100.9 mg of compound I was weighed into a glass bottle, and 31.75 mL of tetrahydrofuran and 2.05 mL of water were added thereto to obtain a clear solution. The solution was filtered, rotary evaporated at 40°C to obtain a solid, the solid was dried in vacuum at 50°C for 16 h, then the resulting solid was heated to 160°C at 10°C/min and kept for 10 min, and then returned to room temperature to obtain a crystalline solid.

Upon detection, the resultant crystalline solid is crystal form CSI of the present invention, its X-ray powder diffraction data are shown in Table 2, and its X-ray powder diffraction pattern is shown in Fig. 1. TGA is as shown in Fig. 2. It has a mass loss of about 0.01% when heated to 100°C. The crystal form CSI is anhydrous.

**Table 2**

| Diffraction angle 20 (°) | d value (Å) | Relative intensity (%) |
|---|---|---|
| 6.5 | 13.7 | 2.2 |
| 7.5 | 11.7 | 100.0 |
| 11.9 | 7.4 | 2.6 |
| 13.0 | 6.8 | 12.0 |
| 13.7 | 6.5 | 14.4 |
| 14.0 | 6.3 | 1.2 |
| 14.7 | 6.0 | 5.3 |
| 15.1 | 5.9 | 10.0 |
| 16.8 | 5.3 | 39.4 |
| 18.9 | 4.7 | 5.6 |
| 19.6 | 4.5 | 2.4 |
| 19.8 | 4.5 | 2.0 |
| 20.5 | 4.3 | 2.7 |
| 20.8 | 4.3 | 2.8 |
| 21.3 | 4.2 | 2.4 |
| 22.0 | 4.0 | 1.1 |
| 22.8 | 3.9 | 7.3 |
| 23.6 | 3.8 | 5.6 |
| 24.9 | 3.6 | 1.5 |
| 25.6 | 3.5 | 9.1 |
| 26.1 | 3.4 | 1.2 |
| 27.8 | 3.2 | 1.4 |
| 28.0 | 3.2 | 4.1 |
| 29.3 | 3.0 | 1.0 |
| 30.1 | 3.0 | 2.5 |

### Example 2: Preparation method for crystal form CSI

13.7 mg of compound I was weighed into a glass bottle, 0.4 mL of benzonitrile was added thereto, the mixture was stirred at room temperature for 3 days, and then the sample was transferred to 50°C and stirred for 3 days. The solid was separated, and vacuum dried at 50°C for 1.5 h to obtain a crystalline solid.

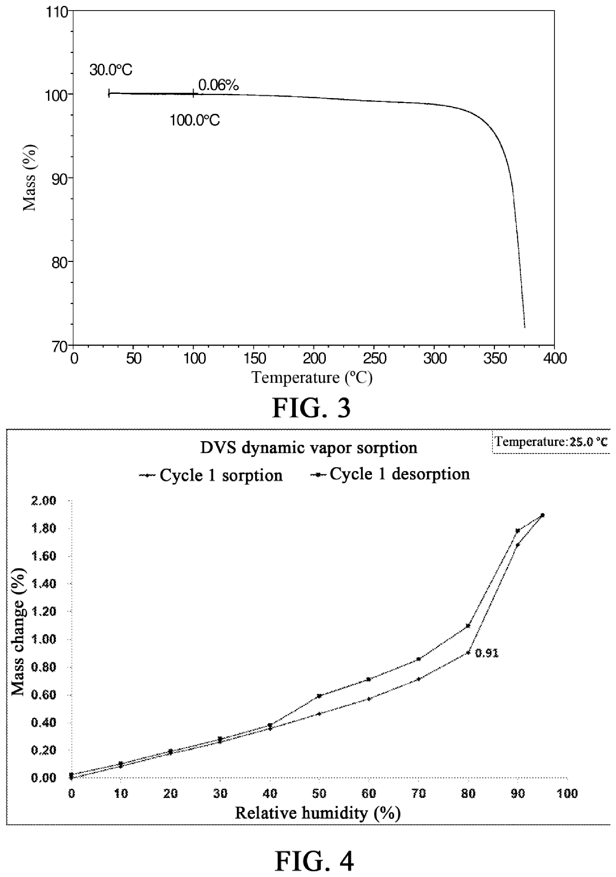

Upon detection, the resulting crystalline solid is crystal form CSI of the present invention, and its X-ray powder diffraction data are shown in Table 3. The TGA diagram is shown in Fig. 3. When heated to 100°C, it has a mass loss of about 0.06%.

**Table 3**

| Diffraction angle 20 (°) | d value (Å) | Relative intensity (%) |
|---|---|---|
| 6.5 | 13.7 | 3.2 |
| 7.5 | 11.7 | 100.0 |
| 11.5 | 7.7 | 12.4 |
| 11.9 | 7.4 | 4.0 |
| 13.0 | 6.8 | 19.6 |
| 13.7 | 6.5 | 26.8 |
| 14.7 | 6.0 | 10.1 |
| 14.9 | 6.0 | 9.5 |
| 15.1 | 5.9 | 10.9 |
| 16.2 | 5.5 | 23.6 |
| 16.8 | 5.3 | 64.4 |
| 17.9 | 4.9 | 3.3 |
| 18.9 | 4.7 | 10.2 |
| 19.8 | 4.5 | 5.0 |
| 20.5 | 4.3 | 7.2 |
| 21.2 | 4.2 | 7.0 |
| 22.5 | 4.0 | 7.1 |
| 22.8 | 3.9 | 10.3 |
| 23.6 | 3.8 | 14.9 |
| 25.6 | 3.5 | 25.3 |
| 28.1 | 3.2 | 10.1 |
| 30.1 | 3.0 | 5.9 |

### Example 3: Hygroscopicity and stability of crystal form CSI

An appropriate amount crystal form CSI of the present invention was tested for hygroscopicity and weight gain by a dynamic vapor sorption instrument. It was circulated once at 0%RH-95%RH-0%RH, and the mass change under each humidity was recorded. The DVS diagram is shown in Fig 4, and the weight gain of crystal form CSI is 0.91% under 80%RH. Comparison of XRPD of crystal form CSI before and after DVS is shown in Fig. 5. The results show that the crystal form has not changed before and after DVS, and crystal form CSI has good humidity stability.

An appropriate amount crystal form CSI prepared by the present invention was stored under 40°C/75%RH and 60°C/75%RH, respectively, and the purity and crystal form were determined by HPLC and XRPD. The results are shown in Table 4, and comparison of XRPD is shown in Fig. 6. The results show that crystal form CSI can remain stable for at least 3 months at 40°C/75%RH and 60°C/75%RH. It can be seen that crystal form CSI can maintain good stability under accelerated and more stringent conditions.

**Table 4**

| Storage conditions | Packaging conditions | Storage time | Crystal form | Purity |
|---|---|---|---|---|
| Starting | - | - | Crystal form CSI | 99.81% |
| 40°C/75%RH | Sealed (wit desiccant) | 3 months | Crystal form CSI | 99.83% |
| | Exposed | | Crystal form CSI | 99.78% |
| 60°C/75%RH | Sealed (wit desiccant) | 3 months | Crystal form CSI | 99.83% |
| | Exposed | | Crystal form CSI | 99.79% |

### Example 4: Preparation method for crystal form CSII

10.8 mg of compound I was weighed into a glass bottle, and 0.7 mL of N,N-dimethylacetamide was added thereto to give a clear solution. The solution was filtered, volatilized at 50°C for 2 days, and then volatilized at 80°C for 1 day to obtain a crystalline solid.

Upon detection, the resulting crystalline solid is crystal form CSII of the present invention, its X-ray powder diffraction data are shown in Table 5, and its X-ray powder diffraction pattern is shown in Fig. 7. TGA is as shown in Fig. 8. It has a mass loss of about 0.04% when heated to 100°C. Crystal form CSII is anhydrous.

**Table 5**

| Diffraction angle 20 (°) | d value (Å) | Relative intensity (%) |
|---|---|---|
| 7.9 | 11.2 | 16.9 |
| 8.6 | 10.2 | 49.6 |
| 10.9 | 8.1 | 28.8 |
| 12.5 | 7.1 | 59.8 |
| 15.9 | 5.6 | 2.1 |
| 17.1 | 5.2 | 7.0 |
| 17.3 | 5.1 | 3.1 |
| 18.1 | 4.9 | 8.2 |
| 19.0 | 4.7 | 1.6 |
| 20.0 | 4.4 | 2.7 |
| 22.0 | 4.0 | 100.0 |
| 24.4 | 3.7 | 1.1 |
| 25.1 | 3.5 | 1.0 |
| 26.1 | 3.4 | 37.4 |
| 27.7 | 3.2 | 4.9 |
| 28.4 | 3.1 | 4.2 |
| 28.8 | 3.1 | 17.2 |
| 31.6 | 2.8 | 2.3 |
| 33.2 | 2.7 | 3.4 |
| 34.0 | 2.6 | 2.5 |
| 35.0 | 2.6 | 1.4 |
| 37.0 | 2.4 | 0.6 |

### Example 5: Hygroscopicity and stability of crystal form CSII

An appropriate amount of crystal form CSII of the present invention was tested for hygroscopicity and weight gain by a dynamic vapor sorption instrument. It was circulated once at 0%RH-95%RH-0%RH, and the mass change under each humidity was recorded. The DVS diagram is shown in Fig. 9, and the weight gain of crystal form CSII is 0.22% under 80%RH. Comparison of XRPD of crystal form CSII before and after DVS is shown in Fig. 10. The results show that the crystal form has not changed before and after DVS, and crystal form CSII has good humidity stability.

An appropriate amount of crystal form CSII prepared by the present invention was stored under 40°C/75%RH and 60°C/75%RH, respectively, and the purity and crystal form were determined by HPLC and XRPD. The results are shown in Table 6, and comparison of XRPD is shown in Fig. 11. The results show that crystal form CSII can remain stable for at least 3 months at 40°C/75%RH and 60°C/75%RH. It can be seen that crystal form CSII can maintain good stability under accelerated and more stringent conditions.

**Table 6**

| Storage conditions | Packaging conditions | Storage time | Crystal form | Purity |
|---|---|---|---|---|
| Starting | - | - | Crystal form CSII | 99.60% |
| 40°C/75%RH | Sealed (wit desiccant) | 3 months | Crystal form CSII | 99.63% |
| | Exposed | | Crystal form CSII | 99.62% |
| 60°C/75%RH | Sealed (wit desiccant) | 3 months | Crystal form CSII | 99.66% |
| | Exposed | | Crystal form CSII | 99.64% |

### Example 6: preparation method for crystal form CSIII

19.8 mg of compound I was weighed into a glass bottle, 2.6 mL of a mixed solvent of dimethyl sulfoxide/water (4:1, v/v) was added thereto, the mixture was heated to obtain a clear solution, and filtered. 1.3 mL of filtrate was added to 3 mL of acetonitrile at 5°C and stirred at 5°C to obtain a crystalline solid.

Upon detection, the resulting crystalline solid is crystal form CSIII of the present invention, its X-ray powder diffraction data are shown in Table 7, and its X-ray powder diffraction pattern is shown in Fig. 12.

**Table 7**

| Diffraction angle 20 (°) | d value (Å) | Relative intensity (%) |
|---|---|---|
| 5.7 | 15.4 | 2.6 |
| 7.4 | 11.9 | 100.0 |
| 8.9 | 9.9 | 1.8 |
| 11.0 | 8.0 | 2.2 |
| 11.5 | 7.7 | 16.3 |
| 13.4 | 6.6 | 8.0 |
| 13.7 | 6.4 | 15.9 |
| 14.9 | 5.9 | 25.9 |
| 15.5 | 5.7 | 1.0 |
| 16.2 | 5.5 | 50.5 |
| 17.2 | 5.1 | 3.6 |
| 18.0 | 4.9 | 4.3 |
| 18.6 | 4.8 | 1.1 |
| 19.9 | 4.5 | 1.5 |
| 20.9 | 4.3 | 2.0 |
| 22.4 | 4.0 | 9.1 |
| 23.1 | 3.9 | 1.0 |
| 24.5 | 3.6 | 1.3 |
| 25.7 | 3.5 | 2.2 |
| 27.1 | 3.3 | 0.7 |
| 28.3 | 3.2 | 2.0 |
| 29.0 | 3.1 | 1.2 |
| 29.7 | 3.0 | 0.7 |
| 32.8 | 2.7 | 0.2 |
| 33.5 | 2.7 | 0.3 |
| 35.0 | 2.6 | 1.5 |
| 35.3 | 2.5 | 1.8 |

### Example 7: Hygroscopicity and stability of crystal form CSIII

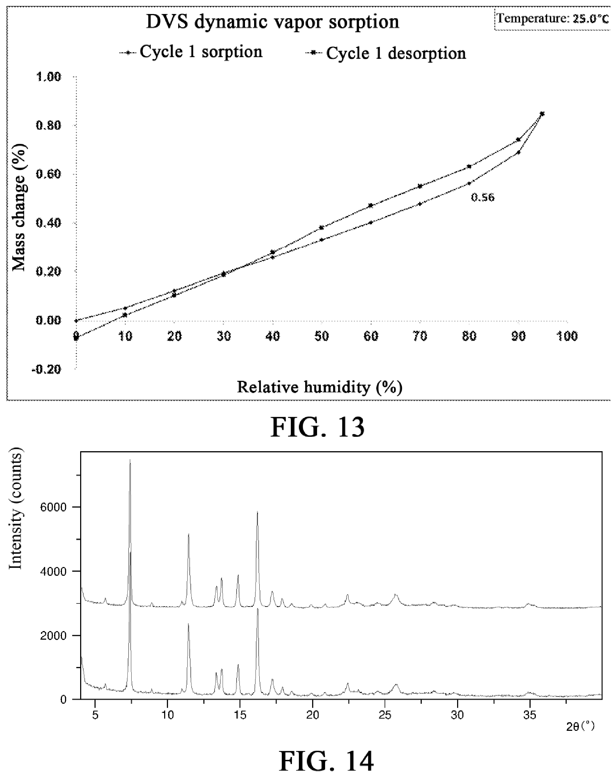

An appropriate amount of crystal form CSIII of the present invention was tested for hygroscopicity and weight gain by a dynamic vapor sorption instrument, it was circulated once at 0%RH-95%RH-0%RH, and the mass change under each humidity was recorded. The DVS diagram is shown in Fig. 13, and the weight gain of crystal form CSIII is 0.56% under 80%RH. Comparison of XRPD of crystal form CSIII before and after DVS is shown in Fig. 14. The results show that the crystal form has not changed before and after DVS, and crystal form CSIII has good humidity stability.

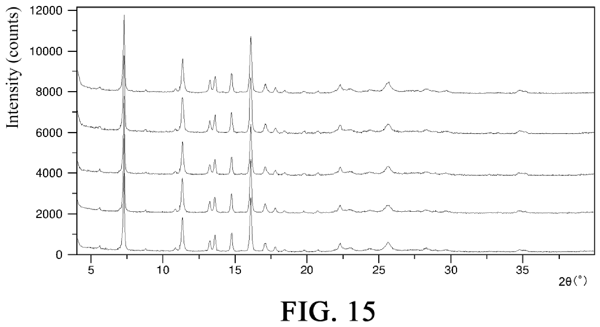

An appropriate amount of crystal form CSIII prepared by the present invention was stored under 40°C/75%RH and 60°C/75%RH, respectively, and the purity and crystal form were determined by HPLC and XRPD. The results are shown in Table 8, and comparison of XRPD is shown in Fig. 15. The results show that crystal form CSIII can remain stable for at least 3 months at 40°C/75%RH and 60°C/75%RH. It can be seen that crystal form CSIII can maintain good stability under accelerated and more stringent conditions.

**Table 8**

| Storage conditions | Packaging conditions | Storage time | Crystal form | Purity |
|---|---|---|---|---|
| Starting | - | - | Crystal form CSIII | 99.76% |
| 40°C/75%RH | Sealed (wit desiccant) | 3 months | Crystal form CSIII | 99.82% |
| | Exposed | | Crystal form CSIII | 99.77% |
| 60°C/75%RH | Sealed (wit desiccant) | 3 months | Crystal form CSIII | 99.82% |
| | Exposed | | Crystal form CSIII | 99.75% |

### Example 8: NMR data of crystal form CSI, crystal form CSII and crystal form CSIII

Crystal form CSI was detected by ¹H NMR to give the following nuclear magnetic data: ¹H NMR (400 MHz, DMSO-d6) δ 12.90 (s, 1H), 12.04 (s, 1H), 9.92 (s, 1H), 8.51 (d, J = 2.1 Hz, 1H), 8.36 (d, J = 2.1 Hz, 1H), 7.93 (d, J = 7.3 Hz, 2H), 7.46 (t, J = 7.7 Hz, 2H), 7.34 (t, J = 7.4 Hz, 1H), 6.91 (d, J = 1.9 Hz, 1H), 2.21 (s, 3H),2.18 (s, 3H).

Crystal form CSII was detected by ¹H NMR to give the following nuclear magnetic data: ¹H NMR (400 MHz, DMSO-d6) δ 12.90 (s, 1H), 12.03 (s, 1H), 9.91 (s,1H), 8.51 (d, J = 2.2 Hz, 1H), 8.35 (d, J = 2.1 Hz, 1H),7.93 (d, J = 7.3 Hz, 2H),7.46 (t, J = 7.7 Hz, 2H), 7.34 (t, J = 7.4 Hz, 1H), 6.91 (d, J = 2.0 Hz, 1H),2.21 (s, 3H), 2.18 (s, 3H).

Crystal form CSIII was tested by ¹H NMR to give the following nuclear magnetic data: ¹H NMR (400 MHz, DMSO-d6) δ 12.91 (s, 1H), 12.05 (s, 1H), 9.94 (s, 1H), 8.51 (d, J = 2.1 Hz, 1H), 8.36 (d, J = 2.1 Hz, 1H), 7.93 (d, J = 7.3 Hz, 2H), 7.46 (t, J = 7.7 Hz, 2H), 7.34 (t, J = 7.4 Hz, 1H), 6.91 (d, J = 1.9 Hz, 1H), 2.21 (s, 3H), 2.18 (s, 3H).

The above-described examples are merely for illustrating the technical concept and features of the present invention, and are intended to enable those skilled in the art to understand the contents of the present invention and carry out the present invention accordingly, and do not limit the scope of protection of the present invention as such. All equivalent changes or modifications made according to the spirit of the present invention should be covered within the scope of protection of the present invention.

## Claims

1. A crystal form of compound I 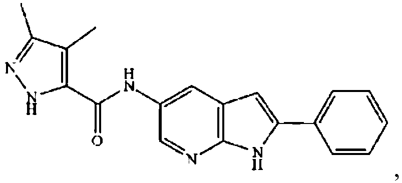 wherein its X-ray powder diffraction pattern by Cu-Kα radiation has characteristic peaks at 20 of 7.5°±0.2°, 13.7°±0.2°, 16.8°±0.2°.

2. The crystal form according to claim 1, wherein its X-ray powder diffraction pattern by Cu-Kα radiation has a characteristic peak at 20 of at least one of 13.0°±0.2°, 15.1°±0.2°, 18.9°±0.2°.

3. The crystal form according to claim 2, wherein its X-ray powder diffraction pattern by Cu-Kα radiation has a characteristic peak at 20 of at least one of 22.8°±0.2°, 23.6°±0.2°, 25.6°±0.2°.

4. The crystal form according to claim 1, wherein its X-ray powder diffraction pattern by Cu-Kα radiation is substantially as shown in Fig. 1.

5. A crystal form of compound I 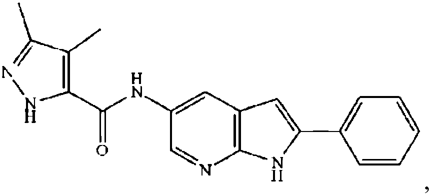 wherein its X-ray powder diffraction pattern by Cu-Kα radiation has characteristic peaks at 2θ of 8.6°±0.2°, 12.5°±0.2°, 22.0°±0.2°.

6. The crystal form of compound I according to claim 5, wherein its X-ray powder diffraction pattern by Cu-Kα radiation has a characteristic peak at 2θ of at least one of 7.9°±0.2°, 10.9°±0.2°, 26.1°±0.2°.

7. The crystal form of compound I according to claim 5, wherein its X-ray powder diffraction pattern by Cu-Kα radiation has a characteristic peak at 20 of at least one of 18.1°±0.2°, 28.8°±0.2°.

8. The crystal form of compound I according to claim 5, wherein its X-ray powder diffraction pattern by Cu-Kα radiation is substantially as shown in Fig. 7.

9. A crystal form of compound I 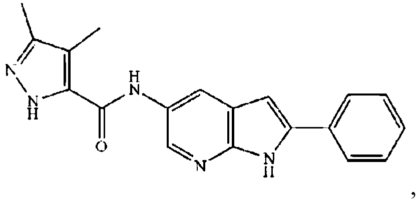 wherein its X-ray powder diffraction pattern by Cu-Kα radiation has characteristic peaks at 20 of 7.4°±0.2°, 11.5°±0.2°,16.2°±0.2°.

10. The crystal form of compound I according to claim 9, wherein its X-ray powder diffraction pattern by Cu-Kα radiation has a characteristic peak at 20 of at least one of 13.4°±0.2°, 14.9°±0.2°, 18.0°±0.2°.

11. The crystal form of compound I according to claim 9, wherein its X-ray powder diffraction pattern by Cu-Kα radiation has a characteristic peak at 20 of 13.7 ±0.2°.

12. The crystal form of compound I according to claim 1, wherein its X-ray powder diffraction pattern by Cu-Kα radiation is substantially as shown in Fig. 12.

13. A pharmaceutical composition, comprising a therapeutically effective amount of the crystal form of claim 1, claim 5 or claim 9, and a pharmaceutically acceptable excipient.

14. Use of the crystal form according to claim 1, claim 5 or claim 9 in preparation of drugs for inhibiting KIT D816V mutations.

15. Use of the crystal form according to claim 1, claim 5 or claim 9 in preparation of drugs for treating gastrointestinal stromal tumor and progressive systemic mastocytosis.
